(19)

Europäisches
Patentamt
European
Patent Office
Office européen
des brevets

(11) **EP 2 243 420 A1**

(12) **EUROPEAN PATENT APPLICATION**

(43) Date of publication:
**27.10.2010 Bulletin 2010/43**

(51) Int Cl.:
**A61B 3/12** (2006.01)  **A61B 5/00** (2006.01)

(21) Application number: **09158781.6**

(22) Date of filing: **24.04.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**

(71) Applicants:
• **Schmidt-Erfurth, Ursula
1090 Wien (AT)**

• **Hitzenberger, Christoph
1090 Wien (AT)**

(72) Inventor: **The designation of the inventor has not yet been filed**

(74) Representative: **Sonn & Partner Patentanwälte
Riemergasse 14
1010 Wien (AT)**

(54) **Method for determining exudates in the retina**

(57) The present invention relates to a method for detecting exudates and/or micro-exudates in the retina of an eye by imaging said retina with a polarisation sensitive optical coherence tomography (OCT) technique comprising the steps of

a) illuminating the retina of said eye with light produced by a polarisation sensitive optical coherence tomography light source,

b) scanning the retina with said light and collecting the reflection light or backscattered light generated in each scanned point in the retina of said eye using signal light collecting means,

c) processing the interferometric signals obtained in at least one polarisation channel, and

d) determining the polarisation properties of said reflection light or backscattered light, and

e) detecting exudates and/or micro-exudates in the retinal layers lying anterior to the retinal pigment epithelium, when the polarisation properties of the reflection light or of the backscattered light of a scanned point deviate from the polarisation properties of light backscattered at structures that are polarisation preserving or birefringent.

Fig. 1

EP 2 243 420 A1

## Description

The present invention relates to a method for determining exudates in the retina.

Diabetic macular edema is a frequent ophthalmologic complication of diabetes mellitus type I and II and known to cause significant visual impairment for the affected patient. A 10-year incidence of diabetic macular edema (DME) with a poor long-term prognosis was found in 13.9% to 25.4% of diabetes patients.

In diabetic macular edema as well as in edema deriving from other pathologic origin, intra-retinal lipid, lipo-protein and macrophage deposits known as "hard exudates" can be observed by biomicroscopy. It has been shown that elevated serum lipid levels are associated with an increased risk of retinal hard exudate in persons with diabetic retinopathy. Although retinal hard exudate usually accompanies diabetic macular edema, increasing amounts of exudate appear to be independently associated with an increased risk of visual impairment. Lowering elevated serum lipid levels has been shown to decrease the risk of cardiovascular morbidity. These data suggest that lipid lowering may also decrease the risk of hard exudate formation and associated vision loss in patients with diabetic retinopathy.

Optical coherence tomography (OCT) gained significant importance throughout the last decade, because it allows evaluating retinal morphology in detail similar to an in vivo histology. However, the conventional OCT imaging is based on 6 radial, cross sectional scans and the information is therefore limited to a few randomly selected locations and an overall low resolution of structural details. The latest OCT generation, spectral domain OCT (SD OCT), uses a fast spectral domain technique and performs scans in a raster pattern throughout the entire macular area, at a superior resolution of $5\mu m$ in axial and $20\mu m$ in transverse direction. As a result, the retinal morphology can be imaged in three dimensions.

A direct differentiation of tissues by backscattered light intensity, as measured by conventional OCT, is often difficult or impossible. However, some tissues can change the polarisation state of backscattered light which can be used as an intrinsic, tissue specific contrast means. Therefore, polarisation sensitive OCT (PS OCT), an extension of the standard OCT technique that uses the polarisation properties of light, can be used to gather additional information on the sample. Several quantities can be retrieved by PS-OCT (e.g. retardation, birefringent axis orientation, Stokes vector). Recent studies have shown that pigmented tissue (e.g. retinal pigment epithelium) depolarises the backscattered light. OCT, as a coherent imaging technique, cannot directly measure the degree of polarisation.

OCT - as mentioned above - can be used to visualise, among others, the retina of the eye, and, thus, serve as a valuable tool in diagnosing an eye disease.

EP 7 000 266 relates to the use of OCT in the diagnosis of retinal eye diseases. The method described in said document comprises the examination of the polarisation properties of the fundus of the eyes.

EP 0 561 643 relates to an OCT imaging device, which can be employed to obtain an image of a living specimen.

In the US 2006/0187462 a method for obtaining OCT data from a sample is described.

It is an object of the present invention to provide a method which allows detecting the presence of exudates and micro-exudates in the retina of the eye of a patient.

The present invention relates to a method for detecting exudates and/or micro-exudates in the retina of an eye by imaging said retina with a polarisation sensitive optical coherence tomography (OCT) technique.

This method preferably comprises the steps of

a) illuminating the retina of said eye with light produced by a polarisation sensitive optical coherence tomography light source,
b) scanning the retina with said light and collecting the reflection light or backscattered light generated in each scanned point in the retina of said eye using signal light collecting means,
c) processing the interferometric signals obtained in at least one polarisation channel, and
d) determining the polarisation properties of said reflection light or backscattered light, and
e) detecting exudates and/or micro-exudates in the retinal layers lying anterior to the retinal pigment epithelium, when the polarisation properties of the reflection light or of the backscattered light of a scanned point deviate from the polarisation properties of light reflected or backscattered by polarisation preserving or birefringent tissue.

Several studies have been performed with the aim to provide a morphological grading of diabetic macular edema based on optical coherence tomography (OCT). However, until now except cyst formation and/or intra-retinal alterations which could be indicative for pathophysiologic processes in diabetic macular edema or edema deriving from other pathologic origin have not been described. It was surprisingly found that intra-retinal precursors of hard exudates, so called micro-exudates, can be detected and visualised with OCT, in particular with spectral domain (SD) OCT and polarisation sensitive OCT. Polarisation sensitive OCT allows to detect these exudates earlier than biomicroscopy. These microexudates are located throughout all retinal layers. If accumulating in the outer retinal layers, they become also visible in fundoscopy as hard exudates.

The micro-exudates surprisingly show depolarising properties, so that they can be detected and/or visualised by using polarising sensitive OCT.

In order to visualise the exudates in an OCT image respective parameters which reflect the depolarisation of the exudates are calculated. A suitable parameter for measuring the amount of depolarisation with PS-OCT is, for instance, the degree of polarisation uniformity (DOPU) which was recently published (E. Götzinger, et al. Optics Express 16(21), 16416-16428 (2008)). DOPU reflects the degree of polarisation by averaging Stokes vector elements within a volume that is larger than the speckle size. With this parameter it is possible to use an automatic segmentation procedure that is based on the intrinsic tissue properties for segmenting depolarising tissue out of the measured volume. In a simplified algorithm, also statistics of the measured retardation and axis orientation values could be used for this purpose. DOPU can be calculated via the Stokes vector S as follows ($A_x$, $A_y$ Amplitudes of the horizontal and vertical polarisation channel, $\Delta\Phi$ phase difference between horizontal and vertical polarisation channel):

$$ S = \begin{pmatrix} I \\ Q \\ U \\ V \end{pmatrix} = \begin{pmatrix} A_x^{\,2} + A_y^{\,2} \\ A_x^{\,2} - A_y^{\,2} \\ 2A_x A_y \cos\Delta\phi \\ 2A_x A_y \sin\Delta\phi \end{pmatrix} $$

To calculate the DOPU values, Stokes vector elements (Q,U,V) are averaged within an evaluation window resulting in $Q_m$, $U_m$, $V_m$.

$$ DOPU = \sqrt{Q_m^2 + U_m^2 + V_m^2} $$

The micro-exudates show markedly decreased DOPU values (0 to 0.8) as compared to other particles within the retina (DOPU = 0.8 to 1). This effect can be used to identify, count and quantify exudates and micro-exudates in the human retina in vivo.

The automated detection, localisation and quantification of intra-retinal exudation in several retinal pathologies is of high value for diagnosis, the follow-up of therapeutic effects and prognostic estimation of the patho-morphologic changes which can be expected over time. It opens new insights into pathologic intra-retinal mechanisms in a very early stage which cannot be assessed by other imaging techniques.

According to a preferred embodiment of the present invention the exudates and/or micro-exudates in the retina are detected by measuring a deviation of polarisation sensitive parameters of the reflection light or of the backscattered light compared to polarisation preserving or birefringent structures of the retina.

The polarisation preserving or birefringent structures of the retina are located in between the retinal pigment epithelium and inner limiting membrane (ILM).

The direction of polarisation may vary for at least 10%, preferably at least 20%, more preferably for at least 40%.

The polarisation sensitive parameters used can be calculated using the Stoke's method (Stoke vectors as described above) or by determining the retardation and/or axis orientation of the sample. Further parameters are Jones matrix elements, Jones vector elements and Mueller matrix elements (see e.g. Serge Huard "Polarization of light" John Wiley&Sons, Chichester/New York/Brisbane/Toronto/Singapore (1997)).

According to a further preferred embodiment of the present invention the signals of step c) are electronically processed to form an image of the retina.

The reflected light from the retina can be processed to an image of the retina. Corresponding methods and computational programmes are known to the person skilled in the art.

According to a preferred embodiment of the present invention the method further comprises the step of visually displaying a depth resolved structure of the retina on the basis of the collected signal data detected.

The method of the present invention allows the detection and the quantification of exudates and micro-exudates in the retina. These deposits precede retinal-associated eye diseases, in particular those eye diseases which are associated with an increased level of serum lipids within the body of an individual. Therefore, the method according to the present invention can be used for diagnosing a retinal-associated eye disease or a risk of developing said eye disease, wherein the presence of exudates and/or micro-exudates in the retina indicates a retinal-associated eye disease or the risk of developing said eye disease.

The method according to the present invention may also be used for monitoring the progress of a retinal-associated eye disease comprising the steps of:

> a) determining the amount of exudates and/or micro-exudates in the retina of an eye of a patient at a first point in time,
> b) determining the amount of exudates and/or micro-exudates in the retina of the eye of said patient at a second point in time, and
> c) comparing the amounts of exudates and/or micro-exudates determined in steps a) and b).

This method can also be used to test medicaments or to monitor their efficacy for treating retinal associated eye diseases. Also the healing process of these diseases can be monitored.

According to a preferred embodiment of the present invention the retinal-associated eye disease is selected from the group consisting of diabetic retinopathy, retinal vessel occlusion and all retinal diseases leading to a break-down of the blood-retina barrier and a consecutive lipid and lipo-protein exudation.

The present invention is further illustrated by the following figures and examples without being restricted thereto.

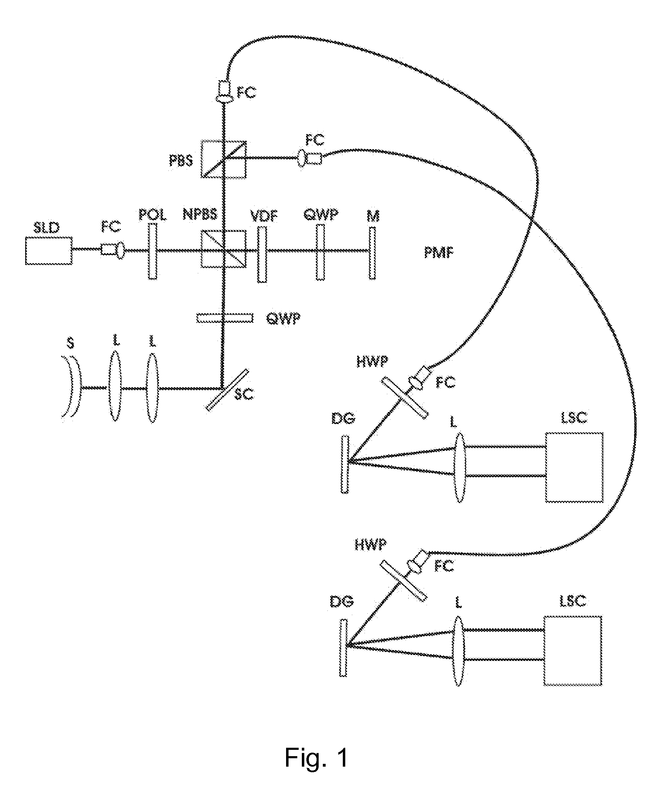

Fig. 1 shows a setup for PS-OCT imaging according to the present invention. SLD, super luminescent diode; FC, fiber coupler; POL, polariser; NPBS, non-polarising beam splitter; VDF, variable density filter; QWP, quarter wave plate; M, mirror; SC, galvo scanner; L, lens; S, sample; PMF, polarisation maintaining fiber; HWP, half wave plate; DG, diffraction grating; LSC, line scan camera.

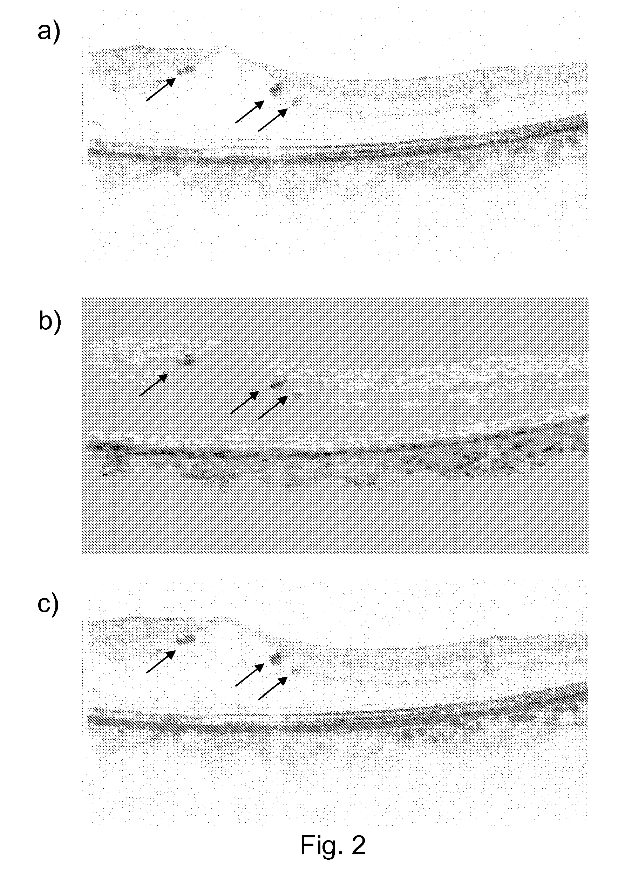

Fig. 2 shows PS-OCT images of a retina with microexudates. Figure 2a shows a SD OCT scan through the foveal center of a patient with diabetic macular edema (standard OCT image). Retinal swelling and intra-retinal cysts are clearly visible, as well as microexudates ( arrows). In figure 2b the polarisation properties of the same retinal area are shown. Figure 2c shows a superimposition of 2a and 2b highlighting the intra-retinal microexudates indicating their characteristic polarisation properties.

**EXAMPLES:**

*Example 1:* Calculation of the degree of polarisation uniformity (DOPU)

An SD PS OCT set-up (Götzinger E, et al. Opt. Express 13, 10217-10229 (2005)) records 20,000 spectra (or A-scans) per second in each polarisation channel. 3D data of the human retina region, covering a scan field of 15° x 15° and consisting of 60 Bscans (1000(x) x 1024 (z) pixels) were acquired within 3 seconds. Imaging depth was ~ 3 mm.

The measured system sensitivity was 98 dB. The theoretic depth resolution within the retina (assuming a refractive index of 1.38) was 4.5 $\mu$m. The instrument needed only one measurement per sample location to retrieve information on intensity, retardation, and axis orientation.

After standard SD-OCT data preprocessing (fixed pattern noise removal, zero padding, and rescaling of the spectral data from wavelength to wave number space) the influence of anterior segment birefringence was compensated. The corrected polarisation data were then used for RPE segmentation. Two different algorithms were used for this purpose. The first, simpler segmentation algorithm was based on the following concept: light backscattered from most of the retinal layers was in a well-defined polarisation state. Only light backscattered from the RPE was depolarised. This was observed in PS-OCT images as a random polarisation state that varies from speckle to speckle, giving rise to randomly varying retardation values.

To avoid erroneous segmentation caused by noise (which also gives rise to random retardation values in PS-OCT imaging), a thresholding procedure based on the intensity data to gate out areas with low signal intensity was applied (i.e., only polarisation data from areas where the intensity exceeds the noise level by a factor of 3 or more was further processed for RPE segmentation; the variation of intensity noise with imaging depth obtained from calibration measurements was taken into account in this procedure). Within the areas where the intensity threshold criterion was met the retardation images were analysed by use of a floating window that moves over the entire image.

A histogram of retardation values within the window for each window position can be calculated. From the histogram, the standard deviation (SD) of the distribution of retardation values can be obtained. If the SD exceeds a certain value, the area in the corresponding window is regarded as displaying a random polarisation state, and the corresponding tissue is classified as RPE.

The second algorithm was based on Stokes vector analysis and has relations to the degree of polarisation DOP. Since OCT is based on a coherent detection method, the light detected from a single speckle was always fully polarised, i.e. DOP = 1 within a single speckle. However, in case of a depolarising layer, the polarisation state of adjacent speckles is uncorrelated, i.e. it varies randomly. This random variation was exploited in an algorithm: After intensity thresholding (see above), the Stokes vector S of each pixel was calculated:

$$S = \begin{pmatrix} I \\ Q \\ U \\ V \end{pmatrix} = \begin{pmatrix} A_x^2 + A_y^2 \\ A_x^2 - A_y^2 \\ 2A_x A_y \cos\Delta\phi \\ 2A_x A_y \sin\Delta\phi \end{pmatrix}$$

where I, Q, U, V denote the four Stokes vector elements, Ax and Ay denote the amplitudes of the two orthogonal polarisation channels x and y, and $\Delta\varphi$ is their phase difference. Then the Stokes vectors were averaged over adjacent pixels by calculating the mean value of each Stokes vector element within a rectangular window (same size as with first algorithm) and a quantity that is called the degree of polarisation uniformity (DOPU) was calculated:

$$DOPU = \sqrt{Q_m^2 + U_m^2 + V_m^2}$$

where the indices m indicate mean Stokes vector elements.

The quantity DOPU has relations to the degree of polarisation DOP well known from polarisation optics, as can be seen by its definition. However, it is emphasised that because of its statistical origin, DOPU can only be derived by local averaging of Stokes vector elements. It might therefore also be regarded as a spatially averaged DOP.

In case of a polarisation preserving or birefringent tissue, the value of DOPU is approximately 1, in case of a depolarising layer, DOPU should be lower than 1. Similar to the first method, a threshold was defined (DOPU = 0.65, empirically determined), where values below this threshold were regarded as depolarised light, and the corresponding tissue was classified as RPE.

*Example 2:*

Patients with diabetic maculopathy showing focal or generalised clinically significant macular edema associated with diabetes mellitus type 2 were examined by PS OCT for imaging structural retinal alteration secondary to diabetic macular edema. PS OCT findings were analysed for microexudates characteristically seen in SD OCT scans as hyper-reflective non-uniform alterations. In SD OCT, microexudates were clearly visible, but did not show characteristic differences in reflectivity compared to other intra-retinal structures or layers (see Fig. 2a). However, polarised light backscattered from these microexudates was depolarised in a characteristical way clearly indicating their intra-retinal location and volume (see Fig. 2b and 2c).

**Claims**

1. Method for detecting exudates and/or micro-exudates in the retina of an eye by imaging said retina with a polarisation sensitive optical coherence tomography (OCT) technique comprising the steps of

   a) illuminating the retina of said eye with light produced by a polarisation sensitive optical coherence tomography light source,
   b) scanning the retina with said light and collecting the reflection light or backscattered light generated in each scanned point in the retina of said eye using signal light collecting means,
   c) processing the interferometric signals obtained in at least one polarisation channel, and
   d) determining the polarisation properties of said reflection light or backscattered light, and
   e) detecting exudates and/or micro-exudates in the retinal layers lying anterior to the retinal pigment epithelium, when the polarisation properties of the reflection light or of the backscattered light of a scanned point deviate from the polarisation properties of light backscattered at structures that are polarisation preserving or birefringent.

2. Method according to claim 1, **characterised in that** the exudates and/or micro-exudates in the retina are detected by measuring a deviation of at least one polarisation parameter of the backscattered light compared to polarisation preserving structures of the retina.

3. Method according to claim 2, **characterized in that** at least one polarisation parameter is selected from the group consisting Stokes vector, Jones vector, amplitude and phase of the reflection light or of the backscattered light.

4. Method according to any one of claims 1 to 3, **characterized in that** the exudates and/or micro exudates in the retina are detected by measuring a deviation of the degree of polarisation uniformity of the reflection light or of the backscattered light compared to polarisation preserving and/or birefringent structures of the retina.

5. Method according to any one of claims 1 to 4, **characterised in that** the signals of step c) are electronically processed to form an image of the retina.

6. Method according to any one of claims 1 to 5, **characterised in that** the method further comprises the step of visually displaying a depth resolved structure of the retina on the basis of the collected signal data detected.

7. Method according to any one of claims 1 to 6 for diagnosing a retinal-associated eye disease or a risk of developing said eye disease, wherein the presence of exudates and/or micro-exudates in the retina indicates a retinal-associated eye disease or the risk of developing said eye disease.

8. Method according to any one of claims 1 to 6 for monitoring the progress of a retinal-associated eye disease comprising the steps of:

   a) determining the amount of exudates and/or micro-exudates in the retina of an eye of a patient at a first point in time,
   b) determining the amount of exudates and/or micro-exudates in the retina of the eye of said patient at a second point in time, and
   c) comparing the amounts of exudates and/or micro-exudates determined in steps a) and b).

9. Method according to any one of claims 1 to 8, **characterised in that** the retinal-associated eye disease is selected from the group consisting of diabetic retinopathy and retinal vessel occlusion.

10. Method according to claim 1, **characterized in that** the exudates and/or micro-exudates in the retina are detected by measuring statistical properties of the polarisation state and/or amplitude and/or phase of the reflection light or the backscattered light.

Fig. 1

a)

b)

c)

Fig. 2

| Europäisches Patentamt European Patent Office Office européen des brevets | **EUROPEAN SEARCH REPORT** | Application Number EP 09 15 8781 |
| --- | --- | --- |

## DOCUMENTS CONSIDERED TO BE RELEVANT

| Category | Citation of document with indication, where appropriate, of relevant passages | Relevant to claim | CLASSIFICATION OF THE APPLICATION (IPC) |
| --- | --- | --- | --- |
| X | US 2007/146632 A1 (CHIPMAN RUSSELL [US]) 28 June 2007 (2007-06-28) <br> * paragraphs [0005] - [0010] * <br> * paragraph [0041] * <br> * paragraphs [0061] - [0095] * <br> * paragraphs [0100] - [0107] * <br> ----- | 1-10 | INV. <br> A61B3/12 <br> A61B5/00 |
| X | US 2008/291463 A1 (MILNER THOMAS E [US] ET AL) 27 November 2008 (2008-11-27) <br> * paragraphs [0037] - [0045] * <br> * paragraphs [0053] - [0056] * <br> * paragraphs [0072] - [0132] * <br> ----- | 1-10 | |
| X | MIURA M. ET AL.: "Imaging Polarimetry in Age-Related Macular Degeneration" INVESTIGATIVE OPHTALMOLOGY & VISUAL SCIENCE, vol. 49, no. 6, June 2008 (2008-06), pages 2661-2667, XP002540983 <br> * the whole document * <br> ----- | 1-8,10 | |
| X | BAUMANN B. ET AL.: "Imaging the human retina in vivo with combined spectral-domain polarization-sensitive optical coherence tomography and scanning laser ophthalmoscopy" PROCEEDINGS OF THE SPIE, vol. 7163, 18 February 2009 (2009-02-18), page 71630N, XP002540984 <br> * the whole document * <br> ----- <br> -/-- | 1-8,10 | TECHNICAL FIELDS SEARCHED (IPC) <br> A61B <br> G01N |

The present search report has been drawn up for all claims

| Place of search | Date of completion of the search | Examiner |
| --- | --- | --- |
| The Hague | 12 August 2009 | Bataille, Frédéric |

EPO FORM 1503 03.82 (P04C01)

Europäisches
Patentamt
European
Patent Office
Office européen
des brevets

# EUROPEAN SEARCH REPORT

Application Number

EP 09 15 8781

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MIURA ET AL: "Imaging Polarimetry in Central Serous Chorioretinopathy" AMERICAN JOURNAL OF OPHTHALMOLOGY, OPHTHALMIC PUBL, CHICAGO, IL, US, vol. 140, no. 6, 1 December 2005 (2005-12-01), pages 1014-1019.e1, XP005221684 ISSN: 0002-9394 * page 1014 - page 1017 * ----- | 1-10 | |
| A | PIRCHER M. ET AL.: "Human Macula Investigated In Vivo with Polarization-Sensitive Optical Coherence Tomography" INVESTIGATIVE OPHTALMOLOGY & VISUAL SCIENCE, vol. 47, no. 12, December 2006 (2006-12), pages 5487-5494, XP002540985 * the whole document * ----- | 1-10 | |

TECHNICAL FIELDS
SEARCHED (IPC)

The present search report has been drawn up for all claims

| Place of search | Date of completion of the search | Examiner |
|---|---|---|
| The Hague | 12 August 2009 | Bataille, Frédéric |

EPO FORM 1503 03.82 (P04C01)

**ANNEX TO THE EUROPEAN SEARCH REPORT
ON EUROPEAN PATENT APPLICATION NO.**

EP 09 15 8781

This annex lists the patent family members relating to the patent documents cited in the above-mentioned European search report.
The members are as contained in the European Patent Office EDP file on
The European Patent Office is in no way liable for these particulars which are merely given for the purpose of information.

12-08-2009

| Patent document cited in search report | Publication date | Patent family member(s) | Publication date |
|---|---|---|---|
| US 2007146632 A1 | 28-06-2007 | NONE | |
| US 2008291463 A1 | 27-11-2008 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 7000266 A
- EP 0561643 A
- US 20060187462 A

**Non-patent literature cited in the description**

- **E. Götzinger et al.** *Optics Express,* 2008, vol. 16 (21), 16416-16428
- **Serge Huard.** Polarization of light. John Wiley&Sons, 1997
- **Götzinger E et al.** *Opt. Express,* 2005, vol. 13, 10217-10229